# EUROPEAN PATENT SPECIFICATION

(11) **EP 2 068 812 B1**
(45) Date of publication and mention of the grant of the patent: **11.04.2012**
(21) Application number: 07828870.1
(22) Date of filing: 21.09.2007
(51) Int. Cl.: A61J 1/00, B65D 81/26, A61K 31/4245

(54) **MEDICINAL PACKAGE**
MEDIZINISCHE VERPACKUNG
EMBALLAGE MÉDICAL

(30) Priority: 25.09.2006 US 846732 P
(43) Date of publication of application: 17.06.2009
(73) Proprietor: Takeda Pharmaceutical Company Limited, Osaka-shi, Osaka 541-0045 (JP)
(72) Inventor: NONOMURA, Koji, Osaka 532-8686 (JP)
(74) Representative: Dossmann, Gérard
(86) International application number: PCT/JP2007/069129
(87) International publication number: WO 2008/041663

(56) References cited:
- EP-A- 0 370 755
- EP-A- 0 466 068
- WO-A-2004/080808
- WO-A-2005/080384
- WO-A-2007/097452

## Description

### TECHNICAL FIELD

The present invention relates to a medicinal package wherein unpleasant smells are reduced.

### BACKGROUND OF THE INVENTION

Among medicinal compounds, some compounds can give out unpleasant smells. For example, captopril, which is an ACE inhibitor, and fursultiamine, which is a vitamin B1 deficiency remedy, give out smells which originate from sulfur compounds. Also, rimatil, which is an antirheumatic, and L-cysteine, which is a liver-function enhancer, give out smells which originate from sulfhydryl group within their molecules. And olmesartan medoxomil, an angiotensin II receptor antagonist, gives out smells which originate from a low molecular carbonyl compound, biacetyl. Unlike the occurrence of side effects, smell is not a fatal property as medicine, but an unpleasant feeling at taking disturbs the convenience as medicine. Especially, in case of medicines which are taken for a long period, such as antidiabetics or antihypertensive drugs, since the patients can feel unpleasant by their smell, it can happen that the patients try to avoid taking the medicine because of the smell. In addition, in clinical trials, an efficacy of a candidate is assessed by comparing its efficacy with a placebo using so-called double blind test, but if the compound to be tested gives out smells, since it becomes easy to distinguish the compound from the placebo, a trouble may be brought about in the double blind trial test. Therefore, preparation of odorous compounds to medicine needs to reduce their smells as much as possible.

Methods for reducing unpleasant smells, generally, include the decomposition method, the absorption method and the masking method. The decomposition method, which is a method wherein odorous materials are decomposed, includes decomposition by ozone, decomposition by a catalyst and decomposition by a chemical agent. The absorption method, which is a method wherein odorous materials are absorbed physically, includes absorption by synthetic zeolite, silica gel, silica-alumina or active carbon, or mixtures of more than two kinds thereof, and absorption to an electric field to which a high voltage is applied. The masking method is a method that makes unpleasant smells less sensible using an aromatic and the like. From the perspective of application to medicinal preparation, it is normally used to reduce smells by making an absorptive removal of odorous materials by packing synthetic zeolite, silica gel, silica-alumina, or active carbon together in a bottle. EP 0 370 755 A1 discloses a medicinal package with a deodorizing agent.

Meanwhile, since some medicinal compounds are unstable to moisture, to prepare a medicine comprising such compounds, a synthetic zeolite, silica gel, silica-alumina, or an active carbon as well as a metallic oxide such as calcium oxide can be used as a desiccant. It is considered that the desiccation by a metallic oxide is caused by a chemical reaction of the metallic oxide with moisture.

### DISCLOSURE OF THE INVENTION

The absorption of moisture or odorous materials to desiccants such as a synthetic zeolite, silica gel, silica-alumina, or an active carbon is a reversible reaction. As the result, concentration level of moisture or smell in the package is under the equilibrium between a headspace in the package and desorption-absorption by the desiccant.

In other words, by change of preservation environment during transportation or storage, removal of smells may become insufficient by desorption of odorous materials from the desiccant. For example, in an environment that exceeds about 50°C, or by change of temperature during transportation by land or by air, moisture or smells, which have been absorbed once, can be desorbed from the desiccant, because of decrease of absorption capacity of the desiccant, which absorbs firstly moisture that exists in the package originally. Further, most of smells insufficiently removed are delivered to a nasal cavity by the moisture in headspace when the package is opened, thereby making the person feel the smells.

The inventor of the present invention dedicatedly researched to realize the efficient and continuous reduction of smell in medicinal preparations comprising compounds giving out unpleasant smells. As a result, the inventor found that a chemical absorption-type desiccant such as a metallic oxide effectively reduced smells in a medicinal preparation, and have completed the present invention.

More specifically, the present invention relates to
(1) a medicinal package comprising a medicinal preparation capable of giving out smells, a packaging component and a chemical absorption-type desiccant wherein the chemical absorption-type desiccant is a metallic oxide;
(2) the package of the aforementioned (1), wherein the metallic oxide is an alkaline earth metallic oxide;
(3) the package of the aforementioned (2), wherein the alkaline earth metallic oxide is calcium oxide;
(4) the package of the aforementioned (1), wherein the chemical absorption-type desiccant is contained in the material composing the packaging component;
(5) the package of the aforementioned (1), wherein the chemical absorption-type desiccant is placed in the internal space formed by the packaging component;
(6) the package of the aforementioned (1), wherein the medicinal preparation capable of giving out smells comprises a compound having a (5-methyl-2-oxo-1,3-dioxol-4-yl)methyl group;
(7) the package of the aforementioned (6), wherein the compound having a (5-methyl-2-oxo-1,3-dioxol-4-yl)methyl group is (5-methyl-2-oxo-1,3-dioxol-4-yl)methyl 2-ethoxy-1-{[2'-(5-oxo-4,5-dihydro-1,2,4-oxadiazol-3-yl)biphenyl-4-yl]methyl}-1H-benzimidazole-7-carboxylate (hereinafter sometimes to be abbreviated as compound A) or (5-methyl-2-oxo-1,3-dioxol-4-yl)methyl 2-cyclopropyl-1-{[2'-(5-oxo-4,5-dihydro-1,2,4-oxadiazol-3-yl)biphenyl-4-yl]methyl}-1H-benzimidazole-7-carboxylate (hereinafter sometimes to be abbreviated as compound B), or a salt thereof;
(9) a method for reducing smells of a medicinal preparation capable of giving out smells, which comprises using a chemical absorption-type desiccant, wherein the chemical absorption-type desiccant is a metallic oxide;
(10) the method of the aforementioned (9), wherein the medicinal preparation capable of giving out smells is preserved in a packaging component, and the chemical absorption-type desiccant is contained in the material composing the packaging component;
(11) the method of the aforementioned (9), wherein the medicinal preparation capable of giving out smells is preserved in a packaging component, and the chemical absorption-type desiccant is placed in the internal space formed by the packaging component; etc.

### BRIEF DESCRIPTION OF THE DRAWINGS

Fig. 1 shows the layer structure of a cold formable laminate for manufacturing blister base parts shown in Fig. 5, corresponding to the section line II-II in Fig. 5.
Fig. 2 shows the layered make-up of a lid film that can be broken open by applying pressure - for the blister base parts according to Fig. 5, along section line III-III in Fig. 5.
Fig. 3 shows the layered make-up of a peelable lid film for blister base parts.
Fig. 4 shows a plan view of a blister base part, made by cold forming the laminate of Fig. 1.
Fig. 5 shows a section through the blister base part of Fig. 4 along line I-I.
Fig. 6 shows a blister base part of Fig. 5 with sealed-on push through film of Fig. 2 or peelable lid film of Fig. 3.

### DETAILED DESCRIPTION OF THE INVENTION

A chemical absorption-type desiccant used in the present invention may be any metallic oxide which can give drying effects by the chemical reaction with moisture, such as an alkaline earth metallic oxide (e.g. calcium oxide (CaO) etc.), an alkaline earth metallic hydroxide (e.g. calcium hydroxide etc.), sulfate of an alkaline earth metal (e.g. magnesium sulfate etc.) and the like.

In the present invention, the chemical absorption-type desiccant may be contained in the material composing the medicinal package, or placed in the internal space of the medicinal package. When the chemical absorption-type desiccant is contained in the material composing the medicinal package, the content of the chemical absorption-type desiccant in the material composing the medicinal package is 0.5 wt.% to 60 wt.%, preferably 5 wt.% to 50 wt.%, more preferably 10 wt.% to 40 wt.%. When the chemical absorption-type desiccant is placed in the internal space of the medicinal package, the amount of the chemical absorption-type desiccant in the internal space of the medicinal package does not have any particular limit, so long as the amount is sufficient to remove the odorous material, that is, sufficient to suppress or reduce the smell. The amount of the chemical absorption-type desiccant can vary depending on kind or shape of the desiccant, distance from the medicinal preparation capable of giving out smells, amount of the compound giving out smells, what the formulation is, volume of the space where the medicinal preparation and the desiccant are placed, amount of the existing or produced odorous material, preservation condition of the medicinal package. For example, when the internal space of the package used in the present invention has a volume of about 200 ml, the amount of the desiccant is about 50 mg to about 100 g, preferably about 300 mg to about 50 g, more preferably about 500 mg to about 20 g.

In the present invention, as a packaging component storing a medicinal preparation that can give out smells, to the extent that the packaging component is something which can store the medicinal preparation that can give out smells in an airtight space, the packaging component is not to be limited to something, but bottles such as a glass bottle, a plastic bottle and the like, packaging bags such as a plastic bag (including those deposited by aluminum, silicon dioxide (silica)), an aluminum laminated bag and the like, a strip package, a metal can and a composite thereof, and a blister package and the like can be used.

In the case where the packaging component is a bottle, the bottle may be composed of uni-layer or multi-layer. When the bottle is composed of uni-layer, it is preferable that the bottle is molded from a resin integrated with a chemical absorption-type desiccant. When the bottle is composed of multi-layer, it is preferable that the outer layer is one having a high barrier property, and the layer containing a chemical absorption-type desiccant is formed by coating, lamination, or integration (e.g., mixing) of the chemical absorption-type desiccant into a resin.

In the case where the packaging component is a blister package, it is desirable that the chemical absorption-type desiccant is contained in the material composing the packaging component by inserting a resin integrated with the chemical absorption-type desiccant in the blister material resin. It is also desirable that outer layer is one having a high barrier property, and that a chemical absorption-type desiccant is contained in materials for the packaging component by being coated or laminated at the inside of the blister material resin, or by directly being integrated (e.g., mixed) with the blister material.

In addition, in the case where the packaging component is a packaging bag or a strip package, it is desirable that the chemical absorption-type desiccant is contained in the material composing the packaging component by inserting a resin integrated with a chemical absorption-type desiccant in the bag or strip packaging material resin. It is also desirable that outer layer is one having a high barrier property, and that a chemical absorption-type desiccant is contained in materials for the packaging component by being coated or laminated at the inside of the bag or strip packaging material resin, or by directly being integrated (e.g., mixed) with the material for the bag or strip package.

The medicinal preparation capable of giving out smells in the present invention can comprise compounds giving out smells themselves, or can comprise compounds giving out smells by their decomposition. There are many compounds which are difficult to distinguish whether they are "compounds giving out smells themselves" or "compounds giving out smells by their decomposition", and such distinction does not have a special meaning in the present invention (hereinafter, "compounds giving out smells themselves" and "compounds giving out smells by their decomposition" will be collectively called "compounds giving out smells").

The compounds giving out smells are, for example, compounds having a (5-methyl-2-oxo-1,3-dioxol-4-yl)methyl group (so-called a medoxomil group) within their molecules (for example, olmesartan medoxomil, compound A, compound B and the like). The compounds having a medoxomil group within their molecules generate a low molecular weight compound, 2,3-butanedione (also called biacetyl or diacetyl), by having their medoxomil ester cleaved gradually, and 2,3-butanedione is considered to be a causative material of the peculiar smell.

Because tautomerism exists in 5-oxo-4,5-dihydro-1,2,4-oxadiazol-3-yl group of the compound A, the compound A is also represented as (5-methyl-2-oxo-1,3-dioxol-4-yl)methyl 2-ethoxy-1-{[2'-(5-oxo-2,5-dihydro-1,2,4-oxadiazol-3-yl)biphenyl-4-yl]methyl}-1H-benzimidazole-7-carboxylate. Likewise, the compound B is also represented as (5-methyl-2-oxo-1,3-dioxol-4-yl)methyl 2-cyclopropyl-1-{[2'-(5-oxo-2,5-dihydro-1,2,4-oxadiazol-3-yl)biphenyl-4-yl]methyl}-1H-benzimidazole-7-carboxylate.

The compound A and the compound B can be manufactured by a method disclosed in the international publication No. 2005/080384 or 2006/107062, or by a method similar thereto.

The compounds giving out smells also may be pharmaceutically acceptable salts thereof. For example, in a case that the compound has an acid functional group, salts with an inorganic base (e.g. alkali metals such as sodium, potassium and the like, alkaline earth metals such as calcium, magnesium and the like, and transition metals such as zinc, iron, copper and the like) or an organic base (e.g. organic amines such as trimethylamine, triethylamine, pyridine, picoline, tromethamine, ethanolamine, diethanolamine, triethanolamine, dicyclohexylamine, t-butylamine, N,N'-dibenzylethylenediamine and the like, and basic amino acids such as arginine, lysine, ornithine and the like) can be used. Also, in a case that a basic functional group is included within the compound, for example, salts with inorganic acids.such as hydrochloric acid, hydrobromic acid, nitric acid, sulfuric acid, phosphoric acid and the like, or salts with organic acids such as acetic acid, phthalic acid, fumaric acid, oxalic acid, tartaric acid, maleic acid, citric acid, succinic acid, methanesulfonic acid, p-toluenesulfonic acid and the like can be used.

The medicinal preparations capable of giving out smells are, for example, solid preparations, which are appropriate for taking orally, such as tablets, capsules, pills and the like.

The solid preparations can be manufactured by a method known per se (e.g. a method written in general rules of medicine manufacture of the 14^{th} revision of Japanese Pharmacopoeia). For example, in case of tablets, effective ingredients and diluents (e.g. lactose, white sugar, glucose, starch, cornstarch, sucrose, microcrystalline cellulose, powdered licorice, mannitol, sorbitol, sodium bicarbonate, calcium phosphate, calcium sulfate and the like) and disintegrating agents (e.g. amino acid, starch, cornstarch, calcium carbonate, carmellose sodium, crosscarmellose calcium, crosscarmellose sodium, low substituted hydroxypropyl cellulose, crospovidone, carboxymethyl starch sodium and the like) are added for mixing, bonding agents (e.g. hydroxypropyl cellulose, hydroxypropyl methylcellulose, polyvinylpyrrolidone, gelatin, starch, gum arabic, tragacanth, carboxy methylcellulose, sodium alginate, pullulan, glycerol and the like) are added to make granules, and lubricants (e.g. magnesium stearate, calcium stearate, refined talc and the like) are added to make tablets. Granules and fine granules are also granulated in an almost same way as that of making tablets, or nonpareil (Product name, spherical granules containing sucrose 75% (W/W) and cornstarch 25% (W/W)) is coated, while being sprayed with water or a solution of a bonding agent such as white sugar, hydroxypropyl cellulose, hydroxypropyl methylcellulose and the like (concentration: about 0.5-70% (W/V)), with a spraying powder containing an effective ingredient and additives (e.g. white sugar, cornstarch, crystalline cellulose, hydroxypropyl cellulose, methylcellulose, polyvinylpyrrolidone and the like) to give granules. In case of capsules, the granules and fine granules are filled in capsules such as gelatin or hydroxypropyl methylcellulose and the like. Or an effective ingredient is filled in capsules such as gelatin, hydroxypropyl methylcellulose and the like along with diluents (e.g. lactose, white sugar, glucose, starch, sucrose, microcrystalline cellulose, powdered licorice, mannitol, sodium bicarbonate, calcium phosphate, calcium sulfate and the like).

The solid preparations can be coated by coating materials for masking, enteric coating, or sustained-release. The coating materials are, for example, hydroxypropyl methylcellulose, ethyl cellulose, hydroxy methylcellulose, hydroxypropyl cellulose, polyoxyethylene glycol, tween 80, pluronic F68, cellulose acetate phthalate, hydroxypropyl methylcellulose phthalate, hydroxy methylcellulose acetate succinate, eudragit (Rohm Co., Ltd., Germany, methacrylic acid·acrylic acid copolymers) and the like, but, if necessary, light screening agents such as titanium dioxide, red iron oxide and the like can be used.

The above and other objects and features of the present invention will become apparent from the following description taken in conjunction with the preferred embodiments thereof with reference to the accompanying drawings, in which:

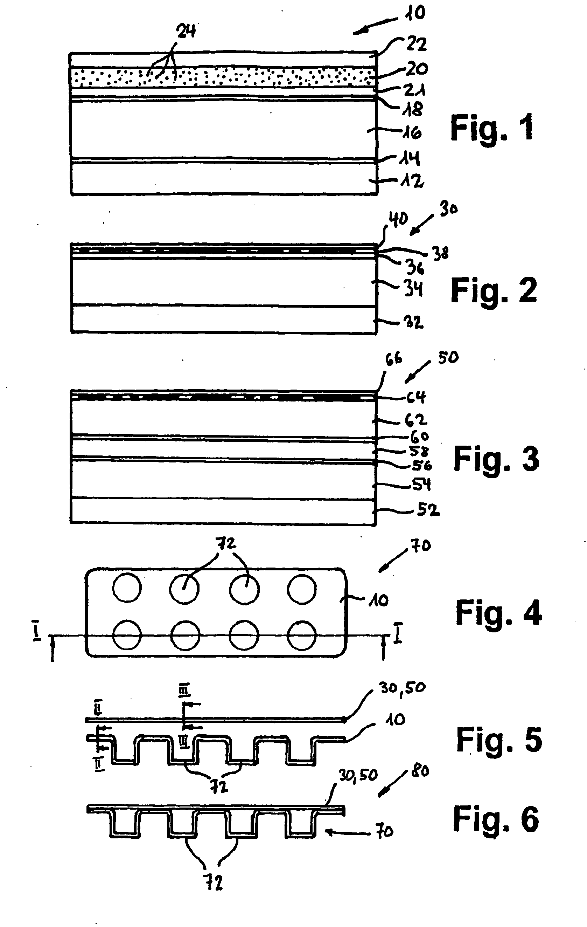

- **Fig. 1**: the layer structure of a cold formable laminate for manufacturing blister base parts shown in Fig. 5, corresponding to the section line II-II in Fig. 5;
- **Fig. 2**: the layered make-up of a lid film - that can be broken open by applying pressure - for the blister base parts according to Fig. 5, along section line III-III in Fig. 5;
- **Fig. 3**: the layered make-up of a peelable lid film for blister base parts;
- **Fig. 4**: plan view of a blister base part, made by cold forming the laminate of Fig. 1;
- **Fig. 5**: section through the blister base part of Fig. 4 along line I-I;
- **Fig. 6**: blister base part of Fig. 5 with sealed-on push through film of Fig. 2 or peelable lid film of Fig. 3.

A cold formable laminate 10 for production of blister base parts for packaging a medicinal preparation capable of giving out smells exhibits, as shown in Fig. 1, the following structure:
12 film of 25 µm thick oriented polyamide (oPA),
14 adhesive layer,
16 45 µm thick aluminium foil,
18 bonding agent (EAA),
21 7 µm thick first layer of high density polyethylene (HDPE),
20 45 µm thick second layer of polyethylene (PE), with
24 30 % CaO-particles as absorbent for moisture, oxygen and acids,
22 7 µm thick third layer of high density polyethylene (HDPE).

The oPA-film 12 forms the layer outer side of a blister made from laminate 10; the PE-layers 20, 21, 22 as sealing layer form the inner side.

A lid film 30 in the form of a push-through film for a blister made from the laminate 10 exhibits, as shown in Fig 2, the following layered structure:
32 hot-sealing lacquer or sealing coating
34 aluminium foil
36 printing pre-lacquer
38 printing
40 printing outer-lacquer

The printing 38 with outer lacquer forms the - later - outer side of lid film 30, the hot-sealing lacquer or sealing coating 32 serves to seal the lid film 30 to the sealing layer 22 of a blister base part made from laminate 10.

A lid film 50, in the form of a peelable film, for a blister base part made from laminate 10 exhibits, as shown in Fig 3, the following layered structure.
52 hot-sealing lacquer or sealing coating
54 aluminium foil
56 adhesive layer
58 polyethylene-terephthalate (PET) film
60 adhesive layer
62 paper
64 printing
66 printing outer-lacquer

The printing 64 with outer lacquer layer 66 forms the - later - outer side of peelable lid film 50, the hot-sealing lacquer or sealing coating 52 serves to seal the lid film 50 to the PE layer 22 of a blister base part made from laminate 10.

A blister base part 70 shown in Fig. 4 is made from the laminate 10, whereby the cups 72 to accommodate e.g. tablets shape-formed in the laminate 10 by cold forming e.g. deep drawing using punch and mould.

As shown in Figs. 5 and 6, after the cups 72 have been filled, a push-through lid film 30 or peelable lid film 50 is sealed onto the blister base part 70 to manufacture a blister pack 80.

The blister base part and the blister pack described in the European patent application No. 05405383 can be preferably used for the present invention.

The inner layer of the blister base part is preferably made of polyolefin and contains as absorbent material preferably an oxide from the group of alkaline and alkaline earth metals. Calcium oxide (CaO) is especially preferred as absorbent material. The preferred CaO content of the polyolefin inner layer is 0.5 to 50 wt.%, in particular 10 to 30 wt.% CaO.

The polyolefin of the inner layer is preferably a high density polyethylene (HDPE) and/or a linear low density polyethylene (LLDPE) and/or a low density polyethylene (LDPE) and/or polypropylene (PP). It may also contain components of acid-modified polyolefins such as ionomers e.g. Surlyn^{®}, EAA or PP-MSA. These acid-modified polyolefins act as bonding agents so that in certain cases a separate primer can be dispensed with. The polyolefin of the inner layer may be of one single layer or several layers.

Especially preferably used in the present invention is a base part of a laminate in which the polyolefin of the inner layer is comprised of at least two layers, whereby the outermost layer, the layer furthest removed from the aluminium foil, contains essentially no absorbent material. This enables a smooth surface to be obtained, with the result that the coefficient of friction of the laminate according to the invention corresponds to that of conventional laminates. By the absence of an additive such as CaO in the outermost layer there is, in comparison with conventional laminates, also no abrasion of shaping tools or other machine components during processing.

Especially preferably used in the present invention is a base part made from a laminate in which the polyolefin of the inner layer is a co-extruded layer of at least two layers, whereby the outermost layer, furthest removed from the aluminium foil, contains essentially no absorbent material.

In the case of the laminate of the base part, the barrier layer is preferably an aluminium foil and is coated on the side facing the polyolefin with a bonding agent, in particular with a water-based or solvent-based primer, or with a polymeric bonding agent.

The innermost layer, lying next to the barrier layer, preferably contains essentially no absorbent material. As a result, during the compressive cold forming process, the particles of absorbent material can not be pressed into the aluminium foil serving as barrier layer. Consequently, no potentially weak points can be created in the aluminium foil and, thereby, also the pore-free formability is not reduced.

The outer layer is preferably a plastic film of oriented polyamide (oPA), oriented polypropylene (oPP) or oriented polyester, which is joined to the aluminium foil via an adhesive layer.

The blister pack used in the present invention has a lid film which is sealed on to the inner side of the blister base part and features a barrier layer as barrier against water vapour and gases and a sealable inner layer on a first side on the barrier layer.

The polyolefin of the inner layer is preferably comprised of one single layer. The sealable inner layer comprises a sealing medium in the form of a lacquer, in particular a hot-sealing lacquer, a film or a sealable coating and serves to seal the lid film to the inner side of the blister base part, whereby the sealing can be a permanent seal or a seal with lower bond strength in order to form a peelable opening.

The barrier layer is preferably an aluminium foil. First, in order to manufacture a blister pack for the purpose of packaging a medicinal preparation capable of giving out smells, a blister base part is made from the laminate by way of cold forming. After filling the blister base part, a lid film containing a barrier layer against water vapour and gasses is sealed onto the inner side of the blister base part.

### EXAMPLE

In the following Example, Comparative Example and Experimental Example, (5-methyl-2-oxo-1,3-dioxol-4-yl)methyl 2-ethoxy-1-{[2'-(5-oxo-4,5-dihydro-1,2,4-oxadiazol-3-yl)biphenyl-4-yl]methyl}-1H-benzimidazole-7-carboxylate potassium salt (hereinafter to be abbreviated as Compound a) was used.

### Example

In the following example, as the components (additive) other than Compound a, those listed in the Japanese Pharmacopoeia, the Japanese Pharmacopoeia quasi drug or the pharmaceutical product additive standard, and the like can be used.

### Formulation A

| | |
|---|---|
| (1) Compound a | 21.34 mg |
| (2) Granulated lactose | 135.1 mg |
| (3) Light anhydrous silicic acid | 0.320 mg |
| (4) Magnesium stearate | 3.200 mg |

Compound a, granulated lactose, light anhydrous silicic acid, and magnesium stearate were mixed and filled in capsules to give a preparation (Formulation A) containing the above-mentioned formula per one capsule.

Formulation A was packed in the space of the blister package containing CaO described above (as shown in Figs. 1, 2 and 4-6).

### Comparative Example

Formulation A was packed in a space of a blister package without desiccant wherein a moisture impermeable packaging film is heat-sealed on a moisture impermeable seal material.

### Experimental Example

After the medicinal packages obtained in the Example and the Comparative Example were preserved under the condition of 25°C-60%RH for six months, or 40°C-75%RH for one, three or six months, concentration of biacetyl, which is one of the odorous components, in the blister packages was measured by gas chromatography.

### Measurement condition of gas chromatography

| | |
|---|---|
| Equipment | : Gas Chromatograph SHIMAZU GC-2010 |
| Detector | : flame ionization detector |
| Column | : SPB-5 (made by SUPELCO; 0.53 mm i.d. x 30 m; membrane thickness 5.0 µm) |
| Column Temp. | : 80°C |
| Carrier Gas | : helium |
| Flow Rate | : 4.5 mL/min |
| Inlet Temp. | : 200°C |
| Detector Temp. | : 260°C |
| Injection Volume : | 0.2 mL |

Results of the concentration (µg/ml) of biacetyl in the blister packages are shown in Table 1 and Table 2.

**Table 1. 25°C-60%RH**

| | Initial | 6 months |
|---|---|---|
| Example | 0 | 0 |
| Comparative Example | 0.00312 | 0.00285 |

**Table 2. 40°C-75%RH**

| | Initial | 1 month | 3 months | 6 months |
|---|---|---|---|---|
| Example | 0 | 0 | 0 | 0 |
| Comparative Example | 0.00312 | 0.00298 | 0 | 0 |

### INDUSTRIAL APPLICABILITY

The medicinal package of the present invention remarkably reduced the concentration of biacetyl under the condition that is commonly used for a stability test of medicines, and maintained the deodorizing activities.

Therefore, the present invention is useful to improve product's value of medicinal preparations capable of giving out smells.

This application is based on U.S. patent application No. 60/846,732.

## Claims

1. A medicinal package comprising a medicinal preparation capable of giving out smells, a packaging component and a chemical absorption-type desiccant, wherein the chemical absorption-type desiccant for reducing smells of said medicinal preparation is a metallic oxide.

2. The package according to Claim 1, wherein the metallic oxide is an alkaline earth metallic oxide.

3. The package according to Claim 2, wherein the alkaline earth metallic oxide is calcium oxide.

4. The package according to Claim 1, wherein the chemical absorption-type desiccant is contained in the material composing the packaging component.

5. The package according to Claim 1, wherein the chemical absorption-type desiccant is placed in the internal space formed by the packaging component.

6. The package according to Claim 1, wherein the medicinal preparation capable of giving out smells comprises a compound having a (5-methyl-2-oxo-1,3-dioxol-4-yl)methyl group.

7. The package according to Claim 6, wherein the compound having a (5-methyl-2-oxo-1,3-dioxol-4-yl)methyl group is (5-methyl-2-oxo-1,3-dioxol-4-yl)methyl 2-ethoxy-1-{[2'-(5-oxo-4,5-dihydro-1,2,4-oxadiazol-3-yl)biphenyl-4-yl]methyl}-1H-benzimidaxole-7-carboxylate or (5-methyl-2-oxo-1,3-dioxol-4-yl)methyl 2-cyclopropyl-1-{[2'-(5-oxo-4,5-dihydro-1,2,4-oxadiazol-3-yl)biphenyl-4-yl]methyy-1H-benzimidazole-7-carboxylate, or a salt thereof.

8. The package according to claim 4, wherein the content of the chemical absorption-type dessicant is 0.5 wt.% to 60 wt.%.

9. A method for reducing smells of a medicinal preparation capable of giving out smells, which comprises using a chemical absorption-type desiccant, wherein the chemical absorption-type desiccant for reducing smells of said medicinal preparation is a metallic oxide.

## Patentansprüche

1. Medizinische Verpackung, welche eine medizinische Zubereitung, die Gerüche abgeben kann, eine Verpackungskomponente und ein chemisches Trockenmittel des Absorptionstyps umfasst, wobei es sich bei dem chemischen Trockenmittel des Absorptionstyps zur Verringerung der Gerüche der medizinischen Zubereitung um ein Metalloxid handelt.

2. Verpackung nach Anspruch 1, wobei es sich bei dem Metalloxid um ein Erdalkalimetalloxid handelt.

3. Verpackung nach Anspruch 2, wobei es sich bei dem Erdalkalimetalloxid um Calciumoxid handelt.

4. Verpackung nach Anspruch 1, wobei das chemische Trockenmittel des Absorptionstyps in dem Material enthalten ist, aus dem die Verpackungskomponente aufgebaut ist.

5. Verpackung nach Anspruch 1, wobei das chemische Trockenmittel des Absorptionstyps in dem Innenraum angeordnet ist, der durch die Verpackungskomponente gebildet wird.

6. Verpackung nach Anspruch 1, wobei die medizinische Zubereitung, die Gerüche abgeben kann, eine Verbindung umfasst, die eine (5-Methyl-2-oxo-1,3-dioxol-4-yl)methyl-Gruppe aufweist.

7. Verpackung nach Anspruch 6, wobei es sich bei der Verbindung, die eine (5-Methyl-2-oxo-1,3-dioxol-4-yl)methyl-Gruppe aufweist, um (5-Methyl-2-oxo-1,3-dioxol-4-yl)methyl-2-ethoxy-1-{[2'-(5-oxo-4,5-dihydro-1,2,4-oxadiazol-3-yl)biphenyl-4-yl]methyl}-1H-benzimidazol-7-carboxylat oder (5-Methyl-2-oxo-1,3-dioxol-4-yl)methyl-2-cyclopropyl-1-{[2'-(5-oxo-4,5-dihydro-1,2,4-oxadiazol-3-yl)biphenyl-4-yl]methyl}-1H-benzimidazol-7-carboxylat oder ein Salz davon handelt.

8. Verpackung nach Anspruch 4, wobei der Anteil des chemischen Trockenmittels des Absorptionstyps 0,5 Gew.-% bis 60 Gew.-% beträgt.

9. Verfahren zum Verringern von Gerüchen einer medizinischen Zubereitung, die Gerüche abgeben kann, welches die Verwendung eines chemischen Trockenmittels des Absorptionstyps umfasst, wobei es sich bei dem chemischen Trockenmittel des Absorptionstyps zur Verringerung der Gerüche der medizinischen Zubereitung um ein Metalloxid handelt.

## Revendications

1. Conditionnement médical comprenant une préparation médicinale capable d'émettre une odeur, un composant de conditionnement et un agent desséchant agissant par absorption chimique, lequel agent desséchant agissant par absorption chimique pour rendre moins forte l'odeur de ladite préparation médicinale est un oxyde de métal.

2. Conditionnement conforme à la revendication 1, dans lequel l'oxyde de métal est un oxyde de métal alcalino-terreux.

3. Conditionnement conforme à la revendication 2, dans lequel l'oxyde de métal alcalino-terreux est de l'oxyde de calcium.

4. Conditionnement conforme à la revendication 1, dans lequel l'agent desséchant agissant par absorption chimique est contenu dans le matériau constituant le composant de conditionnement.

5. Conditionnement conforme à la revendication 1, dans lequel l'agent desséchant agissant par absorption chimique est disposé dans l'espace intérieur formé par le composant de conditionnement.

6. Conditionnement conforme à la revendication 1, dans lequel la préparation médicinale capable d'émettre une odeur comprend un composé qui comporte un groupe (5-méthyl-2-oxo-1,3-dioxol-4-yl)-méthyle.

7. Conditionnement conforme à la revendication 6, dans lequel le composé comportant un groupe (5-méthyl-2-oxo-1,3-dioxol-4-yl)-méthyle est du 2-éthoxy-1-{[2'-(5-oxo-4,5-dihydro-1,2,4-oxadiazol-3-yl)-biphényl-4-yl]-méthyl}-1H-benzimidazole-7-carboxylate de (5-méthyl-2-oxo-1,3-dioxol-4-yl)-méthyle ou du 2-cyclopropyl-1-{[2'-(5-oxo-4,5-dihydro-1,2,4-oxadiazol-3-yl)-biphényl-4-yl]-méthyl}-1H-benzimidazole-7-carboxylate de (5-méthyl-2-oxo-1,3-dioxol-4-yl)-méthyle, ou un sel de l'un de ces composés.

8. Conditionnement conforme à la revendication 4, dans lequel la proportion de l'agent desséchant agissant par absorption chimique vaut de 0,5 % en poids à 60 % en poids.

9. Procédé permettant de rendre moins forte l'odeur d'une préparation médicinale capable d'émettre une odeur, qui comporte l'utilisation d'un agent desséchant agissant par absorption chimique, et dans lequel l'agent desséchant agissant par absorption chimique pour rendre moins forte l'odeur de ladite préparation médicinale est un oxyde de métal.
